# EUROPEAN PATENT APPLICATION

(11) **EP 4 663 119 A1**
(43) Date of publication of application: **17.12.2025**
(21) Application number: 25181355.6
(22) Date of filing: 06.06.2025
(51) Int. Cl.: A61B 5/11, A61B 5/00, A61B 5/02, A61B 5/024, A61B 5/0295, A61B 5/0535

(54) **MONITORING CARDIOVASCULAR HEALTH USING SENSOR DATA**

(30) Priority: 10.06.2024 US 202463658238 P; 07.05.2025 US 202519201404
(71) Applicant: GOOGLE LLC, Mountain View CA 94043 (US)
(72) Inventor: Jung, Seobin, Mountain View, 94043 (US); Thomson, Seamus David, Mountain View, 94043 (US); Pantelopoulos, Alexandros Antonios, Mountain View, 94043 (US); Yuen, Shelten Gee Jao, Mountain View, 94043 (US); Sunden, Lindsey Michelle, Mountain View, 94043 (US); Richards, Peter Winthrop, Mountain View, 94043 (US)
(74) Representative: Marks & Clerk GST

(57) **Abstract**

A computing system, a computer implemented method, and a wearable computing device to determine a cardiovascular response based at least in part on sensor data of a wearable device. For instance, a computing system receives impedance plethysmography (IPG) data generated by an IPG sensor positioned at a lower side of a housing of a wearable device. The computing system receives photoplethysmography (PPG) data generated by a PPG sensor positioned at the lower side of the housing and proximate to the IPG sensor. Then, the computing system determines a cardiovascular response based at least in part on a comparison of the IPG data and the PPG data, such as a peripheral myogenic response.

## Description

### CROSS-REFERENCE TO RELATED APPLICATION

The present application claims the benefit of United States Provisional Application Number 63/658,238 having a filing date of June 10, 2024. Applicant claims priority to and the benefit of the application listed above and incorporates such application herein by reference in its entirety.

### FIELD OF THE INVENTION

The present disclosure relates generally to monitoring cardiovascular health using non-invasive sensor data obtained from one or more sensors of a wearable computing device. More particularly, the present disclosure relates to passively monitoring myogenic responses using sensor data obtained from at least one or more photoplethysmography (PPG) sensors and one or more impedance plethysmography (IPG) sensors on the wearable computing device, where the monitored myogenic responses are indicative of different cardiovascular health indicators.

### BACKGROUND

Peripheral vasoconstriction and vasodilation are directly tied to many aspects of cardiovascular health, and are impacted by diseases including hypertension, atherosclerosis, peripheral artery disease, Raynaud's phenomenon, diabetes mellitus, heart failure, and/or the like, as well as by aging. However, peripheral myogenic responses, such as peripheral vasoconstriction and peripheral vasodilation, are complex physiological processes which are also influenced by a wide range of factors, such as temperature changes, posture, hydration, exercise, stress, and/or the like, which can make it difficult to accurately monitor with wearable, consumer computing devices. For instance, photoplethysmography (PPG) sensors are optical sensors used to measure pulsatile blood flow by measuring changes in blood volume of the superficial vascular bed, but PPG signals are susceptible to degradation during vasoconstriction due to reduced blood flow in the superficial tissues, and are sensitive to motion and device fit, which makes it difficult to isolate the effects of vasoconstriction in PPG data.

Other techniques for monitoring metrics associated with cardiovascular health include electrocardiogram (ECG) techniques, which may be used to monitor electrical activity associated with the heart. However, ECG sensors for wearable, consumer computing devices require users to actively perform a measurement.

As such, a need exists for a system and method for passively monitoring cardiovascular health metrics, such as peripheral myogenic responses, which may be used as indicators for detecting cardiovascular health related conditions.

### SUMMARY OF THE INVENTION

Aspects and advantages of embodiments of the present disclosure will be set forth in part in the following description, or can be learned from the description, or can be learned through practice of the embodiments.

In one aspect, a system for monitoring cardiovascular health. The system may include a housing of a wearable computing device, the housing having an upper side and a lower side, the lower side facing skin of a user when the wearable computing device is worn by the user. The system may further include an impedance plethysmography (IPG) sensor positioned at the lower side of the housing, where the IPG sensor has a pair of excitation electrodes and a pair of sensing electrodes configured to contact the skin of the user, with the IPG sensor being configured to generate IPG data indicative of a voltage passed between the pair of sensing electrodes due to current applied at the pair of excitation electrodes. Moreover, the system may include a photoplethysmography (PPG) sensor positioned at the lower side of the housing and proximate the IPG sensor, where the PPG sensor may include an emitter configured to emit light and a detector configured to detect the light emitted from the emitter, and where the PPG sensor may be configured to generate PPG data indicative of an amount of light detected by the detector. Additionally, the system may include a computing system configured to receive the IPG data, receive the PPG data, and determine a myogenic response based at least in part on a comparison of the PPG data and the IPG data.

In one more aspect, a computer-implemented method that is capable of conducting the functionality described above with respect to the computing system.

In still another aspect, a wearable computing device is provided that is capable of conducting the functionality described above with respect to the computing system.

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with the description, serve to explain the related principles.

### BRIEF DESCRIPTION OF THE DRAWINGS

Detailed discussion of embodiments directed to one of ordinary skill in the art is set forth in the specification, which makes reference to the appended figures, in which:
FIG. 1 illustrates a front perspective view of a wearable computing device on a wrist of a user according to one embodiment of the present disclosure;
FIG. 2 illustrates a rear perspective view of the wearable computing device of FIG. 1 in accordance with aspects of the present disclosure;
FIG. 3 illustrates another rear view of the wearable computing device of FIGS. 1 and 2, particularly illustrating an IPG sensor and a PPG sensor in accordance with aspects of the present disclosure;
FIG. 4 illustrates various components of an example system that can be utilized according to one embodiment of the present disclosure;
FIG. 5 illustrates a schematic diagram of an example set of devices that are able to communicate according to one embodiment of the present disclosure;
FIG. 6 illustrates a graph comparing PPG data to IPG data during a baseline event, a vasoconstriction event, and a vasodilation event according to an example embodiment of the present disclosure;
FIG. 7 illustrates graphs comparing the amplitudes of PPG data and IPG data before and after a vasoconstriction event for multiple test subjects according to an example embodiment of the present disclosure;
FIG. 8 illustrates a graph comparing the changes in peak amplitudes of the PPG data and the IPG data due to the vasoconstriction event in FIG. 7 according to an example embodiment of the present disclosure;
FIG. 9 depicts a flow diagram of an example, non-limiting computer-implemented method for monitoring cardiovascular health of a wearer of a wearable computing device according to example embodiments of the present disclosure; and
FIG. 10 depicts a graph of average heart rate across a day, particularly illustrating nocturnal dipping in accordance with example aspects of the present disclosure.

Reference numerals that are repeated across plural figures are intended to identify the same features in various implementations.

### DETAILED DESCRIPTION

Reference now will be made in detail to embodiments of the invention, one or more examples of which are illustrated in the drawings. Each example is provided by way of explanation of the invention, not limitation of the invention. In fact, it will be apparent to those skilled in the art that various modifications and variations can be made in the present invention without departing from the scope or spirit of the invention. For instance, features illustrated or described as part of one embodiment can be used with another embodiment to yield a still further embodiment. Thus, it is intended that the present invention covers such modifications and variations as come within the scope of the appended claims and their equivalents.

Generally, the present subject matter is directed to using an impedance plethysmography (IPG) sensor of a wearable computing device to monitor cardiovascular health. For instance, the present subject matter is directed to simultaneously monitoring data generated by an IPG sensor and by a photoplethysmography (PPG) sensor of a wearable computing device positioned at a peripheral location (e.g., wrist) to detect an onset of peripheral vasoconstriction or peripheral vasodilation, narrowing or widening, respectively, of arteries.

Peripheral vasoconstriction and vasodilation are directly tied to many aspects of cardiovascular health, and are impacted by diseases including hypertension, atherosclerosis, peripheral artery disease, Raynaud's phenomenon, diabetes mellitus, heart failure, and/or the like, as well as by aging. However, peripheral vasoconstriction and vasodilation are complex physiological processes which are also influenced by a wide range of factors, such as temperature changes, posture, hydration, exercise, stress, and/or the like, which can make it difficult to accurately monitor. For instance, PPG sensors are optical sensors used to measure pulsatile blood flow by measuring changes in blood volume of the superficial vascular bed, but PPG signals are susceptible to degradation during vasoconstriction due to reduced blood flow in the superficial tissues and are sensitive to motion and device fit, which makes it difficult to isolate the effects of vasoconstriction in PPG data.

The inventors have found that IPG sensors, which measure changes in impedance representative of pulsatile changes in blood volume due to expansion and contraction of blood vessels with each heartbeat, are less affected by the effects of vasoconstriction and vasodilation in comparison to PPG sensors. For instance, when peripheral vasoconstriction happens, PPG magnitude decreases relative to a baseline PPG response while IPG magnitude remains substantially constant relative to a baseline IPG response, and similarly, when peripheral vasodilation happens, PPG magnitude increases relative to a baseline PPG response while IPG magnitude remains substantially the same relative to a baseline IPG response. As such, onset of peripheral vasoconstriction or peripheral vasodilation may be detected based on the relative magnitude changes in PPG and IPG sensor data. Thus, by using a combination of PPG data and IPG data from a wearable computing device as described herein, myogenic responses of a user may be passively monitored over time to monitor a user's cardiovascular health. Moreover, monitoring how the relative magnitudes of the IPG and PPG signals change over time may provide health/wellness insights regarding progression of diseases. For example, if a user had clear PPG and IPG signals, but over time the PPG signal starts to diminish relative to IPG, it may be an indication of peripheral blood flow changes, such as those caused by disease(s), which may indicate that further testing is necessary.

In some instances, the data from the PPG and IPG sensors may be used together or with further data to determine one or more other cardiovascular health indicators. For instance, pulse wave analysis (PWA) derived from the PPG and IPG sensor data and optionally, with ballistocardiogram (BCG) data, provides, or at least improves accuracy of, estimation of blood pressure. Similarly, the data from the PPG and IPG sensors may be used with further data (e.g., motion data, temperature data, and/or the like) to determine tolerance to hot/cold, exercise onset/intensity, exercise training affect, posture, hydration, menstrual cycle, diabetes progression, and/or the like.

Once certain cardiovascular health indicators are detected from at least the PPG and IPG sensors, it is useful to notify the wearer of this information, such as via a notification on a display of the wearable computing device or another computing device (e.g., tablet, mobile phone, laptop, personal computer, etc.) so the wearer can be educated and/or make lifestyle, diet, and/or other changes. In some instances, a wearer may be requested to take active measurements, for instance, by taking an electrocardiogram (ECG) reading with the device, taking a core body temperature, and/or the like based on the detected cardiovascular health indicators.

Accordingly, the disclosed devices, systems, and methods allow for passive monitoring of a user's cardiovascular health by using a combination of IPG and PPG data, which may be used to detect a wearer's status regarding different cardiac and age-related conditions in a non-invasive manner, and to make recommendations based on the detected statuses which can result in the wearer making healthy lifestyle, diet, and other changes.

With reference now to the figures, example embodiments of the present disclosure will be discussed in further detail.

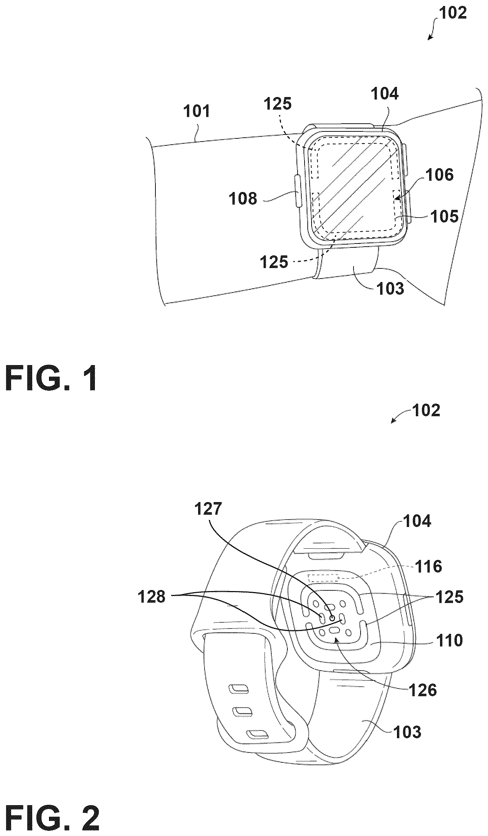

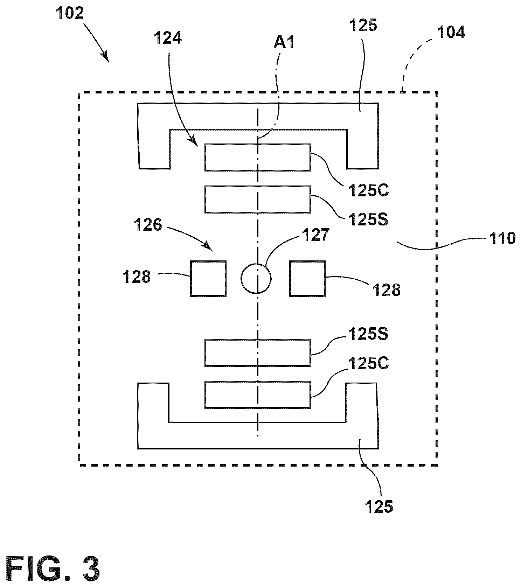

Referring now to the drawings, FIGS. 1-3 illustrate perspective views of a wearable computing device 102 according to the present disclosure. In particular, as shown in FIG. 1, the wearable computing device 102 may be worn on a user or wearer's forearm 101 like a wristwatch. Thus, as shown, the wearable computing device 102 may include a wristband 103 for securing the wearable computing device 102 to the user or wearer's forearm 101. However, it should be appreciated that the wearable computing device 102 may be worn at any other suitable location by a user, such as, for example, on an ankle. It should be further appreciated that the wearable computing device 102 can include a ring, band, earring, necklace, or any other suitable wearable computing device known by one of skill in the art. In addition, as shown in FIGS. 1-3, the wearable computing device 102 has a housing 104 that defines an interior volume for containing electronics associated with the wearable computing device 102. Moreover, the wearable computing device 102 has an outer covering 105 on an upper side for enclosing the interior volume. In an embodiment, the outer covering 105 may be constructed of glass, polycarbonate, acrylic, or similar. Further, as shown in FIG. 1, the wearable computing device 102 includes an electronic display screen 106 arranged within the housing 104 and viewable through the outer covering 105. The electronic display screen 106 may cover an electronics package (not shown), which may also be housed within the housing 104. The display 106 may be any suitable display, such as a touch screen, organic light emitting diode (OLED), or liquid crystal display (LCD). Moreover, as shown, the wearable computing device 102 may also include one or more buttons 108 that may be implemented to provide a mechanism to activate various mechanisms and/or sensors of the wearable computing device 102, such as to collect certain health data of the user. The housing 104 of the wearable computing device 102 further defines a lower side 110 (FIGS. 2 and 3) configured to contact a user (e.g., a dorsal wrist) when being worn by the user.

Referring particularly to FIG. 2, one or more motion sensors 116 may be contained within the housing 104 of the wearable computing device 102 for generating motion data, which can be used, inter alia, to calculate step count, pulse, etc. The motion sensor(s) 116 can include one or more accelerometers for sensing movement data. In some embodiments, the motion sensor(s) 116 can include one or more accelerometers for sensing acceleration or other movement data in each of, for example, three directions (x, y, and z), which may be orthogonal. For instance, the accelerometer can be a triaxial accelerometer. The motion sensor(s) 116 additionally can include one or more gyroscopes for sensing rotation data. In some embodiments, the motion sensor(s) 116 can include one or more gyroscopes for sensing rotation about each of, for example, three axes, which may be orthogonal. The motion sensor(s) 116 additionally can include one or more altimeters, such as a pressure or barometric altimeter. In one or more instances, the motion sensor(s) 116 may include an inertial measurement unit (IMU), which may include a combination of accelerometers and gyroscopes, and/or the like. In some instances, one or more motion sensors 116 (e.g., strain gauges, accelerometers, and/or the like) may be configured as a ballistocardiogram (BCG) sensor for monitoring movement or displacement due to ballistic forces caused by movement of blood with each heartbeat.

Further, the wearable computing device 102 includes one or more photoplethysmography (PPG) sensors 126 disposed proximate the lower side 110 of the housing 104 of the wearable computing device 102 so as to make and maintain skin contact with the user when being worn on the wrist by the user. The PPG sensor(s) 126 has one or more emitters 127, such as one or more light-emitting diodes (LEDs), for emitting controlled pulses of light and has one or more detectors 128, such as photodiodes, to generate data indicative of the detected, returned light. Some PPG technologies rely on detecting light at a single spatial location, adding signals taken from two or more spatial locations, or an algorithmic combination thereof. Both of these approaches result in a single spatial measurement from which the heart rate (HR) estimate (or other physiological metrics) is determined. In some embodiments, the PPG sensor(s) 126 employs a single emitter 127 associated with a single detector 128 (i.e., a single light path). Additionally, or alternatively, the PPG sensor(s) 126 may employ multiple emitters 127 coupled to a single detector 128 or multiple detectors 128 (i.e., two or more light paths). In other embodiments, the PPG sensor(s) 126 may additionally, or alternatively, employ multiple detectors 128 coupled to a single light emitter 127 or multiple emitters 127 (i.e., two or more light paths).

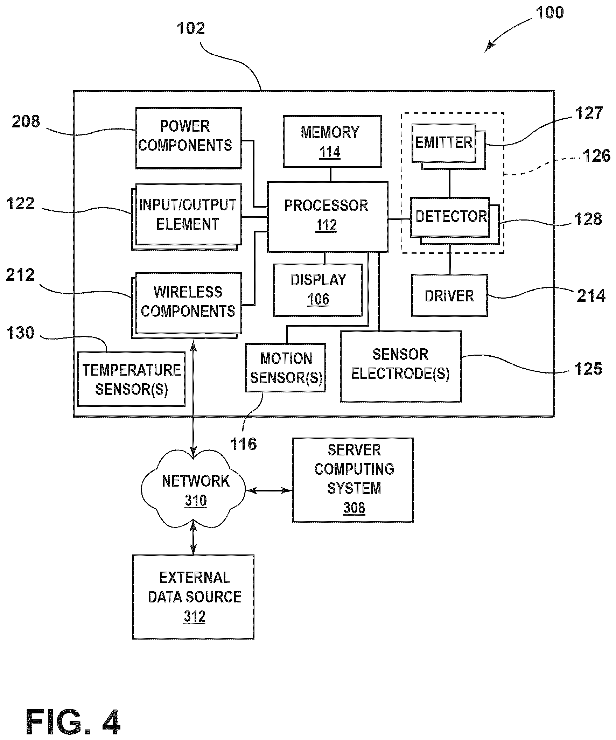

A processor (e.g., processor 112 in FIG. 4) controls the emitter(s) 127 to emit photons, which reflect off the skin, tissue, bones, blood, etc. of a wearer for detection by detectors 128, and converts the analog current received from the detector(s) 128 into a digital PPG signal. Signal changes associated with peripheral perfusion, due to contraction of the heart, enable the wearable computing device to measure the wearer's pulsatility and heart rate, resting heart rate, interbeat interval, heart rate variability, etc. using the PPG signal.

The PPG sensor(s) 126 can be configured for use at various light wavelengths, such as green (centered at 528 nanometers (nm)), red (centered at 660 nm), and infrared (centered at 940 nm), where the amplitude of the reflected light for the green, red, and infrared wavelengths changes with every heartbeat. Typically, the peak to peak amplitude for green PPG signals is greater than the peak to peak amplitude for red PPG signals and infrared PPG signals when there is a clear pulsatile signal such as that associated with a heartbeat. In some cases, a PPG sensor 126 may employ a single light source 127 and two or more light detectors 128 each configured to detect a specific wavelength or wavelength range. In some cases, each detector 128 is configured to detect a different wavelength or wavelength range from the other detectors 128. In other cases, two or more detectors 128 are configured to detect the same wavelength or wavelength range. In yet another case, one or more detectors 128 is configured to detect a specific wavelength or wavelength range different from one or more other detectors 128). In embodiments employing multiple light paths, the PPG sensor(s) 126 may determine an average of the signals resulting from the multiple light paths before determining an HR estimate or other physiological metrics. In any event, the PPG sensor(s) 126 is usable to generate non-invasive biometric data related to resting heart rate, heart rate variability, interbeat interval, etc.

Additionally, the wearable computing device 102 includes a plurality of sensor electrodes 125. For instance, the wearable computing device 102 includes one or more pairs of sensor electrodes 125 on the lower side 110 of the housing 104 so as to maintain skin contact with the user when being worn by the user and configurable to measure, at least, electrical impedance of the user at a location of the skin contact (e.g., on the dorsal wrist), which is associated with electrodermal activity data, and/or the like.

The wearable computing device 102 further includes one or more IPG sensors 124 for generating IPG data, as shown schematically in FIG. 3, where each of the IPG sensors 124 includes at least four of the sensor electrodes 125 positioned on the lower side 110 of the housing 104. For example, the IPG sensor 124 shown in FIG. 3 includes at least one pair of excitation electrodes 125C for providing a stimulating current and at least one pair of sensing electrodes 125S, where the IPG sensor 124 measures the resulting voltage potential across the pairs of voltage sensing electrodes 125S due to the stimulating current applied across the current-injecting, excitation electrodes 125C. In some instances, the sensing electrodes 125S are positioned between the excitation electrodes 125C such that the excitation electrodes 125C are spaced apart by the sensing electrodes 125S. In one or more instances, the IPG sensor 124 includes more than two pairs of sensor electrodes 125C, 125S for generating IPG data. In such instances, the two pairs of sensor electrodes 125C, 125S used to generate IPG data may be selected based at least in part on signal quality of the IPG data and/or proximity to the PPG sensor(s) 126. In some instances, the sensing electrodes 125C, 125S of the IPG sensor(s) 124 are aligned on a common axis A1. For instance, in some embodiments, the sensing electrodes 125 of the IPG sensor(s) 124 are centered on a common axis A1 extending generally parallel to an artery when the wearable computing device 102 is worn. However, in other instances, the sensing electrodes 125C, 125S of the IPG sensor(s) 124 may be positioned in any other suitable pattern.

In particular instances, the PPG sensor(s) 126 are disposed proximate the sensor electrodes 125C, 125S of the IPG sensor(s) 124 to ensure both the PPG sensor(s) 126 and the IPG sensor(s) 124 are generating data indicative for the same anatomical region. For instance, in one embodiment, the PPG sensor(s) 126 is positioned immediately adjacent to one or more of the electrodes 125C, 125S of the IPG sensor(s) 124. In some instances, at least one PPG sensor 126 is positioned between at least some of the electrodes 125C, 125S of the IPG sensor(s) 124. For example, the electrodes 125C, 125S of the IPG sensor(s) 124 may be positioned around the PPG sensor(s) 126, such as with the PPG sensor(s) 126 being between at least one pair of sensing electrodes 125S of the IPG sensor(s) 124. In alternative embodiments, the various components of the PPG sensor 126 may be positioned around the sensor electrodes 125C, 125S of the IPG sensor(s) 124 and/or in another other suitable configuration such as adjacent to, interspersed with, surrounded by, or on below the IPG sensor(s) 124 (e.g., where at least some of the electrodes 125C, 125S are transparent).

As will be described below in greater detail, sensors on the lower side 110 of the housing 104 of the wearable computing device 102 may allow for passive measurements as long as the lower side 110 of the housing 104 is positioned proximate to a user's skin, which may allow for passive measurements of different biometrics.

In one or more instances, the electrodes 125 of the wearable computing device 102 may also include at least one pair of electrodes 125 on the upper side of the wearable computing device 102 for active or on-demand measurement of biometrics (e.g. electrocardiogram (ECG), electrodermal activity (EDA), etc.) of the user wearing the wearable computing device 100. For instance, in some implementations, the user can contact (e.g., touch) the electrodes 125 on the upper side of the wearable computing device 102 where the electrodes 125 on the upper side of the wearable computing device 102 in combination with data from the electrodes 125 on the lower side 110 of the wearable computing device 102 act as an ECG sensor to obtain an on-demand electrocardiogram reading. Alternatively, or additionally, the user can contact (e.g. touch) the electrodes 125 on the upper side of the wearable computing device 102, where the data from the electrodes 125 on the upper side of the wearable computing device 102 is used alone as an EDA sensor to obtain an on-demand electrodermal activity reading. In some implementations, the electrodes 125 on the upper side of the wearable computing device 102 are spaced apart from one another. In some instances, the electrodes 125 on the upper side of the wearable computing device 102 are positioned on an upper surface of the cover 105 and/or wrap around a perimeter of the cover 105. Alternatively, or additionally, the sensor electrodes 125 are positioned at any other suitable location on the wearable computing device 102 such that the sensor electrodes 125 are able to be selectively (e.g., "actively") contacted by a user while wearing the wearable computing device 102 for taking an active measurement.

In some embodiments, the wearable computing device 102 may also include at least one additional biometric sensor electrode in addition to the PPG sensor(s) 126 and the IPG sensor(s) 124. For instance, the wearable computing device 102 may also include one or more temperature sensors 130 (such as an ambient temperature sensor or a skin temperature sensor), a humidity sensor, an ambient light sensor, a pressure sensor, a microphone, another optical sensor (e.g., distance sensor), and/or the like.

Referring now to FIG. 4, components of an example computing system 100 of the wearable computing device 102 that can be utilized in accordance with various embodiments are illustrated. In particular, as shown, the computing system 100 may also include at least one processor 112 communicatively coupled to different parts of the wearable computing device 102, such as the display 106, the motion sensor(s) 116, the sensor electrodes 125 (e.g., EDA electrodes, IPG sensor 124 electrodes 125C, 125S, ECG electrodes, and/or the like), the PPG sensor(s) 126, the temperature sensor(s) 130 (e.g., ambient or skin), and any other sensors present. Moreover, in an embodiment, the processor(s) 112 may be a central processing unit (CPU) or graphics processing unit (GPU) for executing instructions that can be stored in a memory 114, such as flash memory or DRAM, among other such options. For example, in an embodiment, the memory 114 may include RAM, ROM, FLASH memory, or other non-transitory digital data storage, and may include a control program comprising sequences of instructions which, when loaded from the memory 114 and executed using the processor(s) 112, cause the processor(s) 112 to perform the functions that are described herein. As would be apparent to one of ordinary skill in the art, the computing system 100 can include many types of memory, data storage, or computer-readable media, such as data storage for program instructions for execution by any suitable processor. The same or separate storage can be used for images or data, a removable memory can be available for sharing information with other devices, and any number of communication approaches can be available for sharing with other devices.

The computing system 100 also includes one or more power components 208, such as a battery operable to be recharged through conventional plug-in approaches, or through other approaches such as capacitive charging through proximity with a power mat or other such device.

The computing system 100 may also include one or more wireless components 212 operable to communicate with one or more electronic devices within a communication range of the particular wireless channel. The wireless channel can be any appropriate channel used to enable devices to communicate wirelessly, such as Bluetooth, cellular, NFC, Ultra-Wideband (UWB), or Wi-Fi channels. It should be understood that the computing system 100 can have one or more conventional wired communications connections as known in the art.

The computing system 100 may be configured to receive inputs from the display 106 (e.g., when the display 106 is a touch screen) and/or to control the display 106 to convey information, although devices might convey information via other means, such as through audio speakers, projectors, or casting the display or streaming data to another device, such as a mobile phone, wherein an application on the mobile phone displays the data. In further embodiments, the computing system 100 can also include at least one additional input-output (I/O) component 122 able to receive conventional input from a user. This conventional input can include, for example, a push button, touch pad, touch screen, wheel, joystick, keyboard, mouse, keypad, or any other such device or element whereby a user can input a command to the computing system 100. In another embodiment, the I/O component(s) 122 may be connected by a wireless infrared or Bluetooth or other link as well in some embodiments. In some embodiments, the computing system 100 may also include a microphone or other audio capture element that accepts voice or other audio commands. For example, in particular embodiments, the computing system 100 may not include any buttons at all, but might be controlled only through a combination of visual and audio commands, such that a user can control the wearable computing device 102 without having to be in contact therewith. In certain embodiments, the I/O components 122 may also include one or more of the sensor electrodes 125 described herein, optical sensors (e.g., PPG sensor(s) 126), barometric sensors (e.g., altimeter, etc.), temperature sensor(s) 130, and the like.

It should be appreciated that, the emitters 127 and detectors 128 of the PPG sensor(s) 126 may be coupled to the processor 112 directly or indirectly using driver circuitry 214 by which the processor 112 may control the emitters 127 to emit light and obtain signals from the detectors 128. Similarly, the excitation electrodes 125C of the IPG sensor(s) 124 may be coupled to the processor 112 directly or indirectly for supplying current (e.g., from the power component(s) 208) across the excitation electrodes 125C.

Further, a server computing system 308 can communicate with wireless components 212 via one or more networks 310, which may include one or more local area networks, wide area networks, UWB, and/or internetworks using any of terrestrial or satellite links. In some embodiments, the server computing system 308 executes control programs and/or application programs that are configured to perform some of the functions described herein. Moreover, the network(s) 310 may allow one or more other devices(s) to communicate with the wearable computing device 102, such as one or more external data source(s) 312 (e.g., for providing core body temperature data, and/or the like).

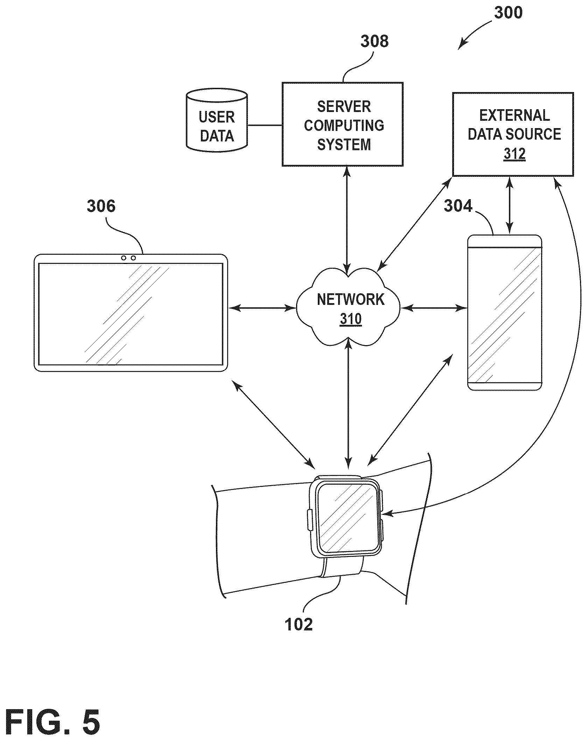

For instance, referring now to FIG. 5, a schematic diagram of an environment 300 in which aspects of various embodiments can be implemented is illustrated. In particular, as shown, a user might have a number of different devices that are able to communicate using at least one wireless communication protocol. For example, as shown, the user might have a wearable computing device 102, such as a smartwatch or fitness tracker, which the user would like to be able to communicate with another device, such as smartphone 304, a tablet computer 306, one or more external data sources 312 (e.g., a thermometer for measuring core body temperature, a scale for measuring weight, a health records system, etc.), and/or the like. The ability to communicate with multiple devices can enable a user to view information from the smartwatch 302, e.g., data captured using a sensor on the smartwatch 302, and any other linked source (e.g., external data source 312) using an application installed on either the smartphone 304 or the tablet computer 306. The user may also want the smartwatch 302 to be able to communicate with the server computing system 308 of the service provider, or other such entity, which is able to obtain and process data from the smartwatch and/or any other suitable device (e.g., the external data source(s) 312) to provide functionality that may not otherwise be available on the smartwatch or the applications installed on the individual devices. In addition, as shown, the smartwatch 302 may be able to communicate with the server computing system 308 of the service provider through the at least one network 310, such as the Internet or a cellular network, or may communicate over a wireless connection such as Bluetooth^{®} to one of the individual devices, which can then communicate over the at least one network.

There may be a number of other types of, or reasons for, communications in various embodiments. For instance, a user or wearer may want to allow the system 100 to access health data from an external health records system (e.g., for a health provider). For instance, the health data can include pre-existing health data of the user or wearer in the form of electronic health records that can include biomarker data, age, pre-existing health conditions, etc. Biomarker data could have been previously collected in an invasive or non-invasive manner and can include biomarkers from blood testing, and this data can be related to a complete blood count, a comprehensive metabolic panel, an insulin level, a blood glucose level, a total cholesterol level, an HDL cholesterol level, an LDL cholesterol level, a triglyceride level, an HbA1c level, a high-sensitivity C-reactive protein level, a gamma-glutamyl transferase level, a testosterone level, a blood urea nitrogen level, a creatinine level, an Estimated Glomerular Filtration Rate (eGFR), a sodium level, a potassium level, a chloride level, a carbon dioxide level, a calcium level, a total protein level, an albumin level, a globulin level, an albumin/globulin ratio, a total bilirubin level, an alkaline phosphatase (ALP) level, an aspartate aminotransferase (AST) level, an alanine aminotransferase (ALT) level, or a combination thereof.

In addition to being able to communicate, a user or wearer may also want the devices to be able to communicate in a number of ways or with certain aspects. For example, the user or wearer may want communications between the devices to be secure, particularly where the data may include personal health data or other such communications. The device or application providers may also be required to secure this information in at least some situations. The user may want the devices to be able to communicate with each other concurrently, rather than sequentially. This may be particularly true where pairing may be required, as the user may prefer that each device be paired at most once, such that no manual pairing is required. The user may also desire the communications to be as standards-based as possible, not only so that little manual intervention is required on the part of the user but also so that the devices can communicate with as many other types of devices as possible, which is often not the case for various proprietary formats. A user may thus desire to be able to walk in a room with one device and have such device automatically communicate with another target device with little to no effort on the part of the user. In various conventional approaches, a device will utilize a communication technology such as Wi-Fi to communicate with other devices using wireless local area networking (WLAN). Smaller or lower capacity devices, such as many Internet of Things (IoT) devices, instead utilize a communication technology such as Bluetooth^{®}, and in particular Bluetooth Low Energy (BLE) which has very low power consumption.

In further embodiments, the environment 300 illustrated in FIG. 5 enables data to be captured, processed, and displayed in a number of different ways. For example, data may be captured using sensors on the smartwatch 302, but due to limited resources on the smartwatch 302, the data may be transferred to the smartphone 304 or the server computing system 308 of the service provider (or a cloud resource) for processing, and results of that processing may then be presented back to that user on the smartwatch 302, smartphone 304, and/or another such device associated with that user, such as the tablet computer 306. In at least some embodiments, a user may also be able to provide input such as health data from an external data source using an interface on any of these devices, which can then be considered when making that determination.

As mentioned above, the data collected from the IPG sensor(s) 124 and the PPG sensor(s) 126 can be utilized in order to passively detect (e.g., without a user having to actively position themselves to measure) one or more cardiovascular health responses for a wearer of the wearable computing device 102. More particularly, data generated by the IPG sensor(s) 124 and by the PPG sensor(s) 126 of the wearable computing device 102 positioned at a peripheral location (e.g., wrist, leg, finger, etc.) may be simultaneously monitored to detect an onset of peripheral vasoconstriction or peripheral vasodilation, narrowing or widening, respectively, of arteries. In general, the IPG sensor(s) 124, which measures changes in impedance representative of pulsatile changes in blood volume, are less affected by the effects of vasoconstriction and vasodilation in comparison to the PPG sensor(s) 126.

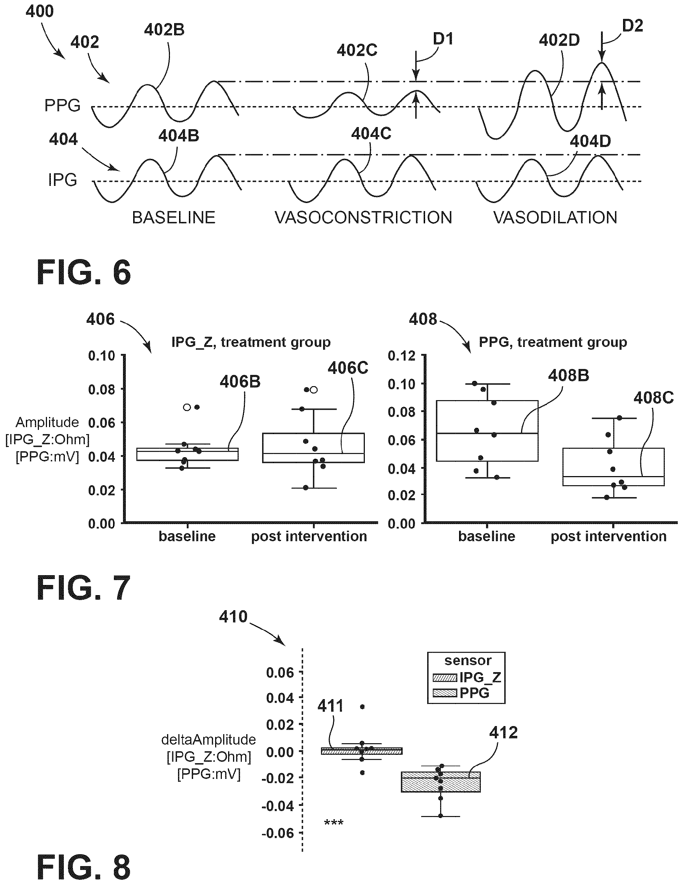

For instance, referring now to FIG. 6, a graph 400 comparing PPG data 402 generated by a PPG sensor 126 to IPG data 404 generated by the IPG sensor(s) 124 is illustrated during a baseline event, a vasoconstriction event, and a vasodilation event according to an example embodiment of the present disclosure. The magnitude of the PPG data 402C during the vasoconstriction event is reduced by a first amount D1 compared to the magnitude of the PPG data 402B during the baseline event, while the magnitude of the IPG data 404C during the vasoconstriction event is substantially the same as the magnitude of the IPG data 404B during the baseline event. Similarly, the magnitude of the PPG data 402D during the vasodilation event is increased by a second amount D2 compared to the magnitude of the PPG data 402B during the baseline event, while the magnitude of the IPG data 404D during the vasodilation event is substantially the same as the magnitude of the IPG data 404B during the baseline event. As such, onset of peripheral vasoconstriction or peripheral vasodilation may be detected based on the relative magnitude changes in PPG and IPG sensor data. Thus, when a magnitude of data from the PPG sensor(s) 126 decreases without a significant, respective change in magnitude in the data from the IPG sensor(s) 124, it may be determined that a vasoconstriction event occurred, or conversely, when a magnitude of data from the PPG sensor(s) 126 increases without a significant, respective change in magnitude in the data from the IPG sensor(s) 124, it may be determined that a vasodilation event occurred. In some embodiments, a myogenic response (e.g., vasoconstriction or vasodilation) is determined only when a magnitude of the PPG data 402 changes by a threshold amount (e.g., the first amount D1 or second amount D2) relative to a change (or lack thereof) in magnitude of the IPG data 404.

FIGS. 7 and 8 show further examples illustrating the relationship between PPG data and IPG data. For instance, FIG. 7 illustrates graphs comparing the peak amplitudes of PPG data and IPG data before a vasoconstriction event (during a baseline event) and after a vasoconstriction event (post intervention) for multiple test subjects and FIG. 8 illustrates a graph comparing the changes in peak amplitudes of the PPG data and the IPG data due to the vasoconstriction event according to an example embodiment of the present disclosure. In the graph 406 in FIG. 7, the mean amplitude 406B of IPG data for the baseline event was essentially the same as the mean amplitude 406C of the IPG data post-intervention (after a vasoconstriction event was caused). Meanwhile, as shown in the graph 408 in FIG. 7, the mean amplitude 408C of the PPG data post-intervention changed (i.e., decreased) from the mean amplitude 408B of the PPG data during the baseline. Correspondingly, in the graph 410 in FIG. 8 for the PPG data, the mean change 412 in amplitude of the PPG data was about negative 20 millivolts [mV], whereas the mean change 411 in amplitude of the IPG data was about 0.002 Ohms. It should be appreciated that the values provided in FIGS. 7 and 8 are for example purposes based on an example sample set to illustrate that PPG data is sensitive (or more sensitive) to myogenic events than IPG data. The relative changes in IPG data and PPG data during use may vary depending on many factors, including, but not limited to, severity of change in environmental temperature, exercise intensity, cardiovascular conditions, and/or the like, as will be described below in greater detail. Corresponding statistical data on the amplitude of the PPG data, the change in amplitude of the PPG data, the amplitude of the IPG data, and the change in amplitude of the IPG data is shown in Table 1 below.

**Table 1: Summary of example statistical effect of intervention on PPG data and IPG data**

| | **Baseline v. Post intervention** | |
|---|---|---|
| **Sensor** | **t-stats** | **p-value (<0.05 is statistically meaningful)** |
| **PPG** | 5.727 | **0.001** |
| **ΔPPG** | 5.853 | **0.001** |
| **IPG** | -0.435 | 0.677 |
| **ΔIPG** | 1.161 | 0.284 |

As shown in Table 1 above, the intervention (e.g., cause of the vasoconstriction event from FIGS. 7-8) was statistically significant (caused a p-value of less than 0.05) for the PPG amplitude (p-value of 0.001), and thus, also for the change in PPG amplitude (p-value of 0.001), but not for the IPG data (p-value 0.677) or change in IPG data (p-value 0.284). As the change in PPG amplitude was statistically significant, but the change in IPG amplitude was not statistically significant (in other words, the IPG amplitude was substantially constant), it can be determined that a myogenic event occurred. It should be appreciated that the values provided in Table 1 are not intended to be limiting, but are examples based on the values in FIGS. 7 and 8, and as such, serve merely as one example illustrating that PPG data is sensitive (or more sensitive) to myogenic events than IPG data.

As such, by using a combination of PPG data and IPG data from a wearable computing device, such as the wearable computing device 102, as described herein, myogenic responses of a user may be passively detected. Moreover, myogenic responses of a user may be passively monitored over a longer period of time (e.g., days, weeks, months, years, etc.) to monitor a user's cardiovascular health. For instance, monitoring how the relative magnitudes of the IPG and PPG signals change over a longer period of time may provide health/wellness insights regarding progression of diseases. For example, if a user had clear PPG and IPG signals, but over a longer period of time the PPG signal starts to diminish relative to IPG, it may be an indication of peripheral blood flow changes, such as those caused by a long-term cause(s) such as a disease(s) and/or age, which may indicate that further testing is necessary.

In some instances, the data from the PPG and IPG sensors 126, 124 may be used together or with further data to determine one or more cardiovascular health indicators. For instance, the data from the PPG and IPG sensors 126, 124 may be used with motion data, temperature data, and/or the like to determine further indicators of health conditions.

For example, vasoconstriction may occur when a user is exposed to a cold environment and vasodilation may occur when a user is exposed to a warm environment, among other things. To confirm whether a myogenic event detected based on the combination of IPG and PPG data resulted at least in part on one or more of an environment exposure, data from an environment and/or skin temperature sensor (e.g., temperature sensor 130 of the wearable computing device 102) may be used to estimate whether a user is exposed to a change in environmental temperature. For instance, if a change in the skin temperature of the user is above a threshold change, and a myogenic response occurs, the myogenic response may be determined to be based at least in part on the environment exposure. If a user frequently experiences a myogenic response when exposed to a change in environment temperature, depending on the degree of severity of the myogenic response and/or the degree of severity of the change in environment temperature, certain conditions, like Raynaud's, may be present. Myogenic responses are usually early and sustained in response to changes in environmental temperature, however myogenic responses attenuate with age. For instance, older users tend to have a delayed myogenic response when exposed to changes in environment temperature in comparison to younger users. As such, if a user experiences a delayed myogenic response when exposed to changes in environmental temperature, age-related thermoregulation may be detected.

Vasoconstriction often occurs when a user begins exercising, to redistribute blood from non-working tissues to active muscles. As a user continues to exercise and begins to warm up, vasodilation may occur to help cool the user down. Generally, the more intense the exercise is (e.g., the more energy the user expends), the higher the internal or core temperature needs to be before a vasodilation begins. As such, by monitoring the time between when a vasoconstriction and subsequent vasodilation occur when exercise is detected (e.g., based on heart rate being above a heart rate threshold, motion data being above a movement threshold, and/or the like), the intensity of the workout may be estimated. Moreover, exercise-trained, physically active people have earlier, and more responsive skin blood flow responses (relative to body temperature) compared to people who are untrained and/or sedentary. Tracking a change in the time between onset of exercise and vasoconstriction and/or a change in the time between vasoconstriction and dilation for a user can be monitored over time (e.g., weeks, months, years, etc.) to assess a user's training or fitness level. In some instances, a core body temperature reading may also be taken (e.g., using an external data source 312, such as a core body temperature sensor), where the change in core body temperature at which myogenic responses occur during exercises may be tracked over time to determine a user's training level. For example, a decrease in temperature at which vasoconstriction occurs over a period (e.g., month) of training may be associated with the training effect being successful (e.g., causing an increase in training or fitness level).

Vasoconstriction level is usable to estimate the number of daily stressors. Daily psychosocial stressor exposure adversely influences microvascular vasoconstrictor function, regardless of the perceived severity or emotional consequences of the stressor exposure. Generally, more stressor events in a short period (e.g., day) lead to a greater degree of vasoconstriction (less blood flow). As such, a momentary stress algorithm may use a measured vasoconstriction level as a variable in addition to other variables (e.g., heart rate, etc.), where greater vasoconstriction may cause a higher stress estimation. The degree of vasoconstriction ("vasoconstriction level") may be determined based at least in part on the change in PPG amplitude relative to the change (or lack thereof) in IPG amplitude. For instance, a plurality of vasoconstriction levels may be established (e.g., minor vasoconstriction, moderate vasoconstriction, high vasoconstriction, extreme vasoconstriction), where each of the plurality of vasoconstriction levels is associated with a respective change in PPG amplitude for a detected vasoconstriction event (e.g., 50% decrease from baseline PPG amplitude, 60% constriction decrease from baseline PPG amplitude, 70% decrease from baseline PPG amplitude, 80% decrease from baseline PPG amplitude, respectively).

Peripheral vasoconstriction and heart rate increases occur when a user is in an upright (orthostatic) posture to help move blood to the brain. As such, detection of vasoconstriction is usable with heart rate monitoring to detect whether a user is in an upright posture. If the wearable computing device 102 is on a peripheral limb (e.g., arm), a hydrostatic effect is caused in underlying vessel pressure due to changes in limb position relative to a user's core (e.g., raising the arm). As such, motion data (e.g., inertial measurement unit readings from motion sensor(s) 116, etc.) could additionally be used with the vasoconstriction and heart rate monitoring for detecting a user's posture.

Detection of vasoconstriction is usable to estimate hydration status (e.g., hydrated or dehydrated). For instance, while a typical user is dehydrated, temporary vasoconstriction happens to maintain blood pressure, which also maintains tissue perfusion pressure, especially that of the brain. Furthermore, delayed onset of vasodilation with increasing internal temperature happens during hypovolemia and hyperosmolality. As such, monitoring a user's core body temperature (e.g., using a core body temperature sensor) with relation to the degree of vasoconstriction and when vasodilation occurs may be used to determine whether a severe hydration issue may be present.

Users with Type 2 diabetes have decreased heat tolerance. Similar to users experiencing dehydration, users with Type 2 diabetes have a delayed internal temperature threshold for onset of vasodilation during whole body heating compared with users without Type 2 diabetes. As such, monitoring progression of delayed vasodilation over time (e.g., weeks, months, years, etc.) can be used to estimate progression of Type 2 diabetes and/or for scoring overall metabolic health. Users with diabetic neuropathy may experience delayed onset of vasoconstriction (e.g., in response to sympathetic activation that would typically cause vasoconstriction and/or increase in heart rate) compared to users without diabetic neuropathy. As such, similarly monitoring progression of delayed vasoconstriction onset over time (e.g., weeks, months, years, etc.) can be used to estimate progression of diabetes and/or for scoring overall metabolic health.

Detection of vasodilation is usable to track menstrual cycles. For instance, core body temperature increases by about 0.5 degrees Celsius in the mid luteal phase and slightly decreases in the preovulatory phase. In response, thermoregulatory control of skin blood flow mirrors these core body temperature shifts. For example, the threshold core temperature for the onset of vasodilation and sweating is higher in the mid luteal phase than in the preovulatory phase. As such, by monitoring core temperature (e.g., using a core body temperature sensor) and/or sweating (e.g., using electrodes 125 of an EDA sensor, such as on the lower side 110 for continuous monitoring) with relation to when vasodilation occurs over time, cyclical patterns may be determined which can be associated with different menstrual phases.

Peripheral vasoconstriction occurs during obstructive sleep apnea events. As such, monitoring for peripheral vasoconstriction during sleep based on the comparison of PPG and IPG data is usable to detect a potential sleep apnea event. Moreover, by monitoring the degree or level of severity of the vasoconstriction, the severity of a sleep apnea event may be detected. The PPG and IPG data may be used in combination with heart rate data, motion data, and/or the like to detect when sleep is occurring and to more positively determine when a sleep apnea event occurs.

PPG data is used in features like heart rate estimation, passive atrial fibrillation detection, and detecting loss of pulse. However, due to many confounding factors (e.g., motion and device fit), there are moments when PPG data has a degraded signal, which leads to false detections of heart rate, atrial fibrillation, loss of pulse, etc. Since IPG data is usable to detect pulse, IPG data is usable as a complementary signal to the PPG data to reduce false detections. IPG signals can also be used as a reference signal for PPG to compensate for motion artifacts or applied pressure on the sensors.

The combination of IPG data and PPG data can be used to provide a more accurate blood pressure estimation. For instance, arterial stiffness changes occur due to coronary artery disease, aging, metabolic diseases, sleep disturbances, smoking, poor cardiovascular health, and/or the like. An augmentation index value is determined by pulse wave analysis (PWA) and is used as a measure of arterial stiffness. For instance, augmentation index values are typically determined as a ratio of late systolic pressure to early systolic pressure, or as a difference between late and early systolic pressures. There is a strong correlation between an augmentation index value obtained from IPG and blood pressure, where different correlations between augmentation index values and blood pressure are established for different groups (e.g., those with coronary artery disease, those without confirmed coronary artery disease, and/or the like). As such, by determining an augmentation index value from a PWA derived from both IPG data and PPG data, blood pressure may be more accurately estimated based on a known correlation.

Pulse transit time (PTT) derived from collocated and simultaneously acquired BCG data and IPG data, and optionally PPG data, may similarly provide an improved blood pressure estimation. For instance, PTT is the time delay for the pressure wave to travel between two arterial sites and is estimated based on the timing between proximal and distal arterial waveforms, where PTT is often inversely related to BP. By determining the PTT based on IPG data in addition to BCG data and/or PPG data, the PTT may be more accurately determined as the IPG data is less sensitive to other factors (e.g., motion, myogenic responses, and/or the like) compared to BCG and PPG data.

Once certain cardiovascular health indicators are detected from at least the PPG and IPG sensors, it is useful to notify the wearer of this information, such as via a notification on the display 106 and/or through another output device of the wearable computing device 102 or another computing device (e.g., tablet, mobile phone, laptop, personal computer, etc.) so the wearer can be educated and/or make lifestyle, diet, and/or other changes. For instance, notifications indicating myogenic occurrence tracking, environmental heat/cold tolerance (and optionally, potential underlying causes, Raynaud's, age, etc.), exercise tracking, fitness level, and/or exercise impact on fitness level, daily stress estimation, hydration status, Type 2 diabetes risk, menstrual cycle tracking (e.g., estimated menstrual cycle phase), number and/or severity of sleep apnea events (and optional recommendation to talk with physician if number and/or severity are over a threshold), heart rate, atrial fibrillation detection, loss of pulse detection, blood pressure (and optionally, meaning of blood pressure reading and/or changes), and/or the like. In some instances, a user may interact with a conversational interface (e.g., chatbot), which may have access to an artificial intelligence (AI) model (e.g., a machine learned, large language model, and/or the like), via one or more user interfaces (e.g., of the wearable computing device 102, a laptop, a tablet, and/or the like) for a user to ask questions regarding cardiovascular health metrics and receive feedback from the interface. In instances where an AI model is accessible, the feedback may be more personalized based on the user's personal metrics depending on user permission settings.

In some instances, a notification may request a user take active measurements, for instance, by taking an electrocardiogram (ECG) reading with the wearable computing device 102, taking a core body temperature with a core body temperature sensor, and/or the like based on the detected cardiovascular health indicators. For example, as discussed above, ECG with the wearable computing device 102 requires users to actively intervene to perform a measurement (i.e. placing their fingertips or palm onto the electrodes 125 on the upper side of the wearable computing device 102) to facilitate measurement inclusive of the heart. There are developing relationships between ECG readings and IPG signal morphologies, which correlate the electrical processes of the heart with mechanical processes of the heart. For instance, while IPG primarily measures a waveform representative of the mechanical aspect of cardiovascular health, if there are electrical changes to the heart (such as that due to disease), these changes are also reflected in the peripheral mechanical signal detected by IPG. As IPG is a passive measurement, if a morphological change was passively detected in IPG data (alone or in conjunction with other data, such as PPG data), a user may be prompted to take a spot-checking ECG reading to confirm the morphological change. As such, IPG use may be used to prompt for ECG at significant times, which allows ECG to be more useful than random spot-checking by a user.

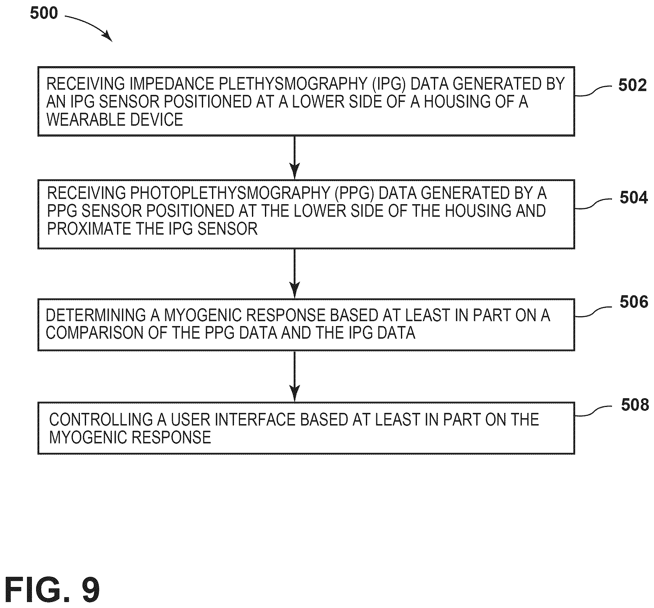

Referring now to FIG. 9, a flow diagram of one embodiment of a method 500 for monitoring cardiovascular health is provided. In general, the method 500 is described herein with reference to the wearable computing device 102 described in FIGS. 1-5 and the graphs of FIGS. 6-8. However, it should be appreciated that the disclosed method 500 may be implemented with any other suitable wearable computing device having any other suitable configurations. In addition, although FIG. 9 depicts steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, added, and/or adapted in various ways without deviating from the scope of the present disclosure.

At step (502), the method 500 may include receiving impedance plethysmography (IPG) data generated by an IPG sensor positioned at a lower side of a housing of a wearable computing device. For instance, as described above, the computing system 100 may receive the IPG data generated by an IPG sensor 124 positioned at the lower side 110 of the housing 104 of the wearable computing device 102, the IPG sensor 124 having a pair of excitation electrodes 125C and a pair of sensing electrodes 125D, the IPG data being indicative of a voltage passed between the pair of sensing electrodes 125S due to current applied at the pair of excitation electrodes 125C and conducted through a user.

The method 500 may further include, at step (504), receiving photoplethysmography (PPG) data generated by a PPG sensor positioned at the lower side of the housing and proximate the IPG sensor. As discussed above, the computing system 100 may receive the PPG data generated by a PPG sensor 126 positioned at the lower side 110 of the housing 104 and proximate the IPG sensor 124, where the PPG sensor 126 includes an emitter 127 configured to emit light and a detector 128 configured to detect the light emitted from the emitter 127, and where the PPG data is indicative of the amount of light detected by the detector 128.

Moreover, the method 500 may further include, at step (506), determining a myogenic response based at least in part on a comparison of the PPG data and the IPG data. For instance, as described above, the computing system 100 may determine a myogenic response (e.g., a vasoconstriction or a vasodilation) based at least in part on a comparison of the PPG data and the IPG data. For example, if an amplitude of the PPG data significantly changes from a baseline PPG amplitude but an amplitude of the IPG data at a corresponding time does not significantly change from a corresponding IPG amplitude, a myogenic response is determined.

Additionally, the method 500 may further include, at step (508), controlling a user interface based at least in part on the myogenic response. For instance, as discussed above, the computing system 100 may control a user interface, such as the electronic display screen 106 of the wearable computing device 102, a screen of another device (e.g., of the smartphone 304, tablet device 306, etc.) or another output element 122 (e.g., speakers, lights, etc.) based at least in part on the myogenic response. For example, the computing system 100 may control the user interface to indicate the myogenic response or a report associated with monitored myogenic responses, such as exercise onset and/or intensity, heat/cold tolerance, diabetes risk, menstrual cycle tracking, sleep apnea detection, cardiovascular health (e.g., arterial stiffness estimation), and/or the like, and/or a request to perform an action, such as taking an ECG measurement with the wearable computing device 102.

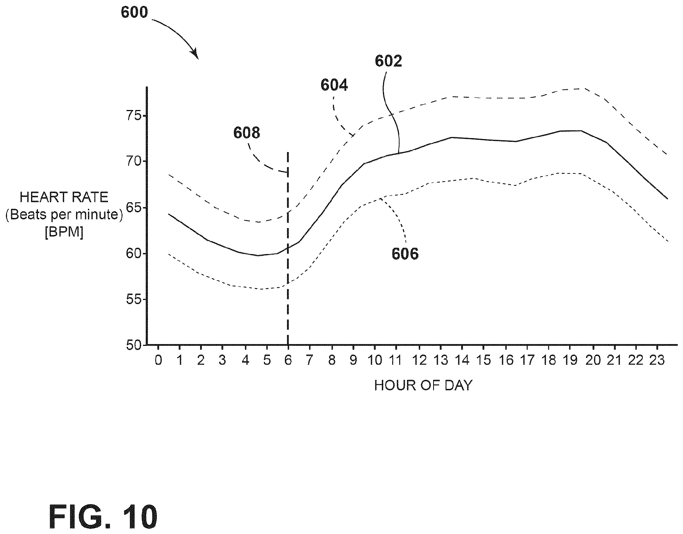

According to further aspects of the present subject matter, heart rate and blood pressure may be monitored (together or separately) for evaluation of nocturnal dipping. Nocturnal dipping is a physiological phenomenon where blood pressure decreases by more than about 10% compared to daytime values. In some instances, heart rate may show a similar nocturnal dip as blood pressure. For example, as shown in FIG. 10, a graph 600 of heart rate across a day for a plurality of test subjects is illustrated. As shown in the graph 600, an average heart rate 602 for the plurality of subjects is plotted in beats per minute (BPM) alongside standard deviation plots 604, 606 across an entire day (e.g., with hour 0 referring to midnight, hour 12 referring to noon, etc.). As shown in the graph 600, the average heart rate 602 (and corresponding standard deviation plots 604, 606) is lowest or "dips" in early morning hours at time 608, between about 4 AM and about 5 AM. The heart rate 602 at time 608 is about 60 BPM, while an average for daytime (e.g., between about 7AM and 9PM) may be about 71BPM, which equates to a nocturnal dip of over a 10% drop in heart rate compared to daytime values.

Some people are of a dipping phenotype, which experience such nocturnal dipping, however, others may be of a non-dipping phenotype, which do not experience such nocturnal dipping. For instance, some users may be double-dipping nocturnal phenotypes, where nocturnal heart rate and nocturnal blood pressure both dip. Some users may be single-dipping nocturnal phenotypes, where heart rate dips without blood pressure dipping or blood pressure dips without heart rate dipping. Some users may be non-dippers, where neither heart rate nor blood pressure dip. Double-dipping users have more positive health outcomes compared to single-dipping users, and single-dipping users generally have better health outcomes compared to non-dipping users. For example, double-dipping users may live longer than single-dipping users, and single-dipping users may live longer than non-dipping users. Further, non-dipping phenotypes may be more likely to experience sleep disorders, such as obstructive sleep apnea, narcolepsy, and/or the like.

Thus, it may be helpful to monitor blood pressure and/or heart rate for nocturnal dipping. Similar to PPG readings, nocturnal blood pressure dip can be affected in the short term by changes in circadian rhythm, autonomic nervous system responses (e.g., stress, sickness, etc.), water and sodium regulation (e.g., hydration level) and/or the like. As such, monitoring nocturnal dipping across several weeks may enable more accurate classification of a user's phenotype. For instance, in some embodiments, heart rate may be monitored over a period of time (e.g., two or more weeks) based on PPG data (e.g., from PPG sensors 126 in FIGS. 2-4), and/or the like to establish whether a user experiences nocturnal dipping in heart rate. In some instances, if the PPG data is low (e.g., as discussed above regarding vasoconstriction and vasodilation) and/or if it is desirable to lessen visible light (e.g., during night hours), IPG data (e.g., from IPG sensor(s) 124 in FIGS. 3-4) may instead, or additionally, be used to monitor heart rate. Additionally, or alternatively, blood pressure may be monitored over a similar period based on PPG data, IPG data, and/or BCG data (e.g., from motion sensors 116 in FIG. 4) to establish whether a user experiences nocturnal dipping in blood pressure. If changes in nocturnal dips in heart rate and/or blood pressure occur over time, there may be significant changes in a user's health, particularly cardiovascular health. In some instances, the computing system 100 may control a user interface associated with the wearable computing device 102 to indicate or report nocturnal dipping phenotype (e.g., double-dipping, single-dipping, non-dipping, etc.), information associated with the phenotype, changes in nocturnal dipping patterns, information associated with changes in nocturnal dipping patterns, and/or the like, and/or recommendations based on phenotype or changes in phenotype (e.g., talk with a doctor, etc.).

### Additional Disclosure

The technology discussed herein makes reference to servers, databases, software applications, and other computer-based systems, as well as actions taken and information sent to and from such systems. The inherent flexibility of computer-based systems allows for a wide variety of possible configurations, combinations, and divisions of tasks and functionality between and among components. For instance, processes discussed herein can be implemented using a single device or component or multiple devices or components working in combination. Databases and applications can be implemented on a single system or distributed across multiple systems. Distributed components can operate sequentially or in parallel.

In addition, although the figures and description depict and describe steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, added, and/or adapted in various ways without deviating from the scope of the present disclosure.

Further to the descriptions above, a user or wearer may be provided with controls allowing the user or wearer to make an election as to both if and when systems, programs, or features described herein may enable collection of user information (e.g., information about a user's health data, activities, social network, social actions, profession, a user's preferences, or a user's current location, etc.), and if the user is sent content or communications from a server. In addition, certain data may be treated in one or more ways before it is stored or used, so that personally identifiable information is removed. For example, a user's identity may be treated so that no personally identifiable information can be determined for the user, or a user's geographic location may be generalized where location information is obtained (such as to a city, ZIP code, or state level), so that a particular location of a user cannot be determined. Thus, the wearer or user may have control over what information is collected about the user, how that information is used, and what information is provided to the user.

While the present subject matter has been described in detail with respect to various specific example embodiments thereof, each example is provided by way of explanation, not limitation of the disclosure. Those skilled in the art, upon attaining an understanding of the foregoing, can readily produce alterations to, variations of, and equivalents to such embodiments. Accordingly, the subject disclosure does not preclude inclusion of such modifications, variations and/or additions to the present subject matter as would be readily apparent to one of ordinary skill in the art. For instance, features illustrated or described as part of one embodiment can be used with another embodiment to yield a still further embodiment. Thus, it is intended that the present disclosure covers such alterations, variations, and equivalents.

## Claims

1. A system for monitoring cardiovascular health, the system comprising:
a housing of a wearable computing device, the housing having an upper side and a lower side, the lower side facing skin of a user when the wearable computing device is worn by the user;
an impedance plethysmography (IPG) sensor positioned at the lower side of the housing, the IPG sensor having a pair of excitation electrodes and a pair of sensing electrodes configured to contact the skin of the user, the IPG sensor being configured to generate IPG data indicative of a voltage passed between the pair of sensing electrodes due to current applied at the pair of excitation electrodes;
a photoplethysmography (PPG) sensor positioned at the lower side of the housing and proximate the IPG sensor, the PPG sensor including an emitter configured to emit light and a detector configured to detect the light emitted from the emitter, the PPG sensor being configured to generate PPG data indicative of an amount of light detected by the detector; and
a computing system configured to:
receive the IPG data;
receive the PPG data; and
determine a myogenic response based at least in part on a comparison of the PPG data and the IPG data.

2. The system of claim 1, wherein the computing system is configured to determine the myogenic response when a magnitude of the PPG data changes by a threshold amount relative to a change in magnitude of the IPG data.

3. The system of claim 1, wherein the computing system is configured to determine the myogenic response by determining a vasoconstriction when a magnitude of the PPG data decreases while a magnitude of the IPG data remains substantially constant and by determining a vasodilation when the magnitude of the PPG data increases while the magnitude of the IPG data remains substantially constant, optionally wherein the computing system is further configured to determine stress of the user based at least in part on determining that the myogenic response includes the vasoconstriction.

4. The system of claim 1, wherein the computing system is further configured to determine a long-term cause when a magnitude of the PPG data decreases relative to a magnitude of the IPG data over a period of time.

5. The system of claim 1, wherein the computing system is further configured to:
receive temperature data generated by a temperature sensor and indicative of at least one of an environmental temperature or a skin temperature; and
determine whether the myogenic response resulted at least in part on one or more of an environment exposure based at least in part on the temperature data, optionally wherein the computing system is configured to determine that the myogenic response resulted at least in part on the environment exposure when a change in the temperature of the user is above a threshold change.

6. The system of claim 1, further comprising a motion sensor within the housing, the motion sensor being configured to generate motion data indicative of movement of the housing,
wherein the computing system is further configured to:
receive the motion data; and
determine an onset of exercise when the myogenic response includes a vasoconstriction, and the motion data is above a movement threshold, optionally wherein the computing system is further configured to determine an intensity of the exercise based at least in part on a time until the myogenic response includes a vasodilation after the vasoconstriction.

7. The system of claim 1, wherein the computing system is further configured to:
receive core body temperature data for the user associated with the wearable computing device; and
determine based at least in part on the myogenic response and the core body temperature data at least one of hydration status, menstrual cycle phase, or training effect for the user.

8. The system of claim 1, further comprising a motion sensor within the housing, the motion sensor being configured to generate motion data indicative of movement of the housing,
wherein the computing system is further configured to:
receive the motion data; and
determine at least one of a sleep apnea event or posture of the user based at least in part on the myogenic response and the motion data.

9. The system of claim 1, wherein the pair of excitation electrodes are spaced apart by the pair of sensing electrodes.

10. The system of claim 1, wherein the PPG sensor is positioned between the pair of sensing electrodes of the IPG sensor.

11. A wearable computing device, comprising:
a housing having an upper side and a lower side, the lower side facing skin of a user when the wearable computing device is worn by the user;
an impedance plethysmography (IPG) sensor positioned at the lower side of the housing, the IPG sensor having a pair of excitation electrodes and a pair of sensing electrodes configured to contact the skin of the user, the IPG sensor being configured to generate IPG data indicative of a voltage passed between the pair of sensing electrodes due to current applied at the pair of excitation electrodes;
a photoplethysmography (PPG) sensor positioned at the lower side of the housing and proximate the IPG sensor, the PPG sensor including an emitter configured to emit light and a detector configured to detect the light emitted from the emitter, the PPG sensor being configured to generate PPG data indicative of an amount of light detected by the detector; and
a computing system configured to:
receive the IPG data;
receive the PPG data; and
determine a cardiovascular response based at least in part on a comparison of the IPG data and the PPG data.

12. The wearable computing device of claim 11, wherein the cardiovascular response comprises at least one of a myogenic response, blood pressure, heart rate, atrial fibrillation, loss of pulse, or nocturnal dipping phenotype.

13. A method for monitoring cardiovascular health, the method comprising:
receiving, with a computing device, impedance plethysmography (IPG) data generated by an IPG sensor positioned at a lower side of a housing of a wearable computing device, the lower side of the housing facing skin of a user when the wearable computing device is worn by the user, the IPG sensor having a pair of excitation electrodes and a pair of sensing electrodes configured to contact the skin of the user, the IPG sensor being configured to generate IPG data indicative of a voltage passed between the pair of sensing electrodes due to current applied at the pair of excitation electrodes;
receiving, with the computing device, photoplethysmography (PPG) data generated by a PPG sensor positioned at the lower side of the housing and proximate the IPG sensor, the PPG sensor including an emitter configured to emit light and a detector configured to detect the light emitted from the emitter, the PPG data being indicative of an amount of light detected by the detector;
determining, with the computing device, a myogenic response based at least in part on a comparison of the PPG data and the IPG data; and
controlling, with the computing device, a user interface based at least in part on the myogenic response.

14. The method of claim 13, wherein determining the myogenic response comprises determining the myogenic response when a magnitude of the PPG data changes by a threshold amount relative to a change in magnitude of the IPG data, or wherein the determining the myogenic response comprises determining the myogenic response by determining a vasoconstriction when a magnitude of the PPG data decreases while a magnitude of the IPG data remains substantially constant and by determining a vasodilation when the magnitude of the PPG data increases while the magnitude of the IPG data remains substantially constant.

15. The method of claim 13, further comprising determining, with the computing device, a long-term cause when a magnitude of the PPG data decreases relative to a magnitude of the IPG data over a period of time, optionally wherein controlling the user interface comprises controlling the user interface to display a recommendation to take an electrocardiogram (ECG) measurement with an ECG sensor of the wearable computing device.
